# EUROPEAN PATENT SPECIFICATION

(11) **EP 2 373 321 B1**
(45) Date of publication and mention of the grant of the patent: **16.03.2016**
(21) Application number: 08876114.3
(22) Date of filing: 05.12.2008
(51) Int. Cl.: A61K 35/19, A61P 17/00, A61K 9/06

(54) **PLATELET LYSATE AND BIOADESIVE COMPOSITIONS THEREOF FOR THE TREATMENT OF MUCOSITIS**
THROMBOZYTEN-LYSAT UND BIOADHÄSIVE ZUSAMMENSETZUNGEN DAVON ZUR BEHANDLUNG VON MUCOSITIS
LYSAT DE PLAQUETTES ET COMPOSITIONS BIOADHÉSIVES DE CELUI-CI POUR LE TRAITEMENT DE LA MUCOSITE

(43) Date of publication of application: 12.10.2011
(73) Proprietor: Biomed Device S.r.l., 50066 Reggello (FI) (IT)
(72) Inventor: CARAMELLA, Carla Marcella, I-27100 Pavia (IT); BONFERONI, Maria Cristina, I-27100 Pavia (IT); ROSSI, Silvia Stefania, I-27100 Pavia (IT); SANDRI, Giuseppina, I-27100 Pavia (IT); FERRARI, Franca, I-27100 Pavia (IT); PEROTTI, Cesare Giuseppe, I-27100 Pavia (IT); DEL FANTE, Claudia, I-27100 Pavia (IT)
(74) Representative: Brunacci, Marco
(86) International application number: PCT/IT2008/000744
(87) International publication number: WO 2010/064267

(56) References cited:
- EP-A- 1 972 685
- WO-A-90/07931
- WO-A-2007/149902
- STACEY M C ET AL: "Randomised double-blind placebo controlled trial of topical autologous platelet lysate in venous ulcer healing" EUROPEAN JOURNAL OF VASCULAR AND ENDOVASCULAR SURGERY, SAUNDERS, LONDON, GB, vol. 20, no. 3, 1 September 2000 (2000-09-01), pages 296-301, XP009122530 ISSN: 1078-5884 [retrieved on 2002-03-25]
- RANZATO E ET AL: "Platelet lysate stimulates wound repair of HaCaT keratinocytes." THE BRITISH JOURNAL OF DERMATOLOGY SEP 2008, vol. 159, no. 3, September 2008 (2008-09), pages 537-545, XP002545178 ISSN: 1365-2133
- MIYAZONO K ET AL: "Platelet-derived endothelial cell growth factor" PROGRESS IN GROWTH FACTOR RESEARCH, PERGAMON PRESS, GB, vol. 3, no. 3, 1 January 1991 (1991-01-01), pages 207-217, XP023260897 ISSN: 0955-2235 [retrieved on 1991-01-01]

## Description

The present invention finds application in the field of medicine; more specifically, it concerns compositions for use in the prevention and treatment of mucositis and of corneal lesions.

### STATE OF THE ART

In many studies, both *in vitro* and *in vivo,* it has been demonstrated how effective growth factors are in promoting cellular proliferation and differentiation, chemiotaxis and synthesis of the extracellular matrix involved in the tissue healing process. Such factors have also been delivered in different release systems. For example, porous three-dimensional polymeric matrices based upon copolymers of lactide and glycolide (PLG) have been proposed to deliver angiogenic factors like the endothelial vascular growth factor (M.H. Sheridan et al., Bioabsorbable polymer scaffolds for tissue engineering capable of sustained growth factor delivery, J. Controlled Release 64, 91- 102 (2000*)*). Gelatine-based biodegradable hydrogels have been studied for the release of the transforming growth factor (TGF-β1) in order to promote bone regeneration (M. Yamamoto et al., Bone regeneration by transforming growth factor b1 released from a biodegradable hydrogel, journal of Controlled Release 64, 133- 142 (2000*)*). Other authors have studied the interaction of the basic fibroblast growth factor (bFGF) with biodegradable hydrogels of gelatine (Y. Tabata et al., In vitro sorption and desorption of basic fibroblast growth factor from biodegradable hydrogels, Biomaterials 19, 1781- 1789 (1998*)*).

Often, the success of the therapy greatly depends upon the location and the anatomical-physiological characteristics of the site covered by the lesion, which, for example, can be difficult to reach or subject to removal mechanisms of the therapeutic agent.

This problem occurs, for example, in the case of mucositis, for which no entirely satisfactory therapeutic agent has yet been described.

Mucositis are lesions that can occur following therapeutic treatments like, for example, radio- and chemo- therapy in cancer treatment. Such mucositis can greatly influence the quality of life with symptoms often so serious as to require, in the case of anti-tumour therapy, that the therapy be halted or shortened or that the drug doses be reduced. It may also become necessary to have parenteral analgesia, hospitalisation and, in the case of buccal mucositis, parenteral nutrition.

The compounds for therapy used up to now have proven to have little activity, are difficult to apply or else, once applied, easily move away or are removed from the site, for example, by physiological secretions or by swallowing movements in the case of buccal mucositis.

Consequently, the active ingredients stay in contact with the mucosa to be treated for an insufficient time, thus slowing down the healing process.

Indeed, the therapeutic effect is not carried out apart from to a minimal extent and, because the first symptoms of healing can occur, it is necessary to perform a continuous and repeated application of the therapeutic agent, which is not always possible, is inconvenient and often unacceptable to the patient.

Similar problems are also encountered in the case of corneal lesions; indeed, the site involved is difficult to reach and the residency time of the active ingredient, often cannot be ensured for a long time period due to lachrymal secretions.

Therefore, therapeutic compositions are necessary comprising an agent or a *pool* of effective therapeutic agents and that are delivered equally effectively to treat mucositis and/or corneal lesions.

### OBJECT OF THE INVENTION

The present invention concerns a pharmaceutical composition comprising a platelet lysate and a carrier for use according to claim 1.

### DETAILED DESCRIPTION OF THE FIGURES

Figure 1 shows the viscosity profiles observed for the carrier 1 at time 0 and after one month of storage at 4-8°C (average values ± SD; n=3).
Figure 2 shows the values of the "maximum adhesion force" parameter evaluated for just the carrier and for the composition 1 (lysate: carrier weight ratio 1:1, see Example 1) (average values ± SD; n=9).
Figure 3 shows the values of the "maximum adhesion force" parameter evaluated for just the carrier and for the composition 2 (lysate: carrier weight ratio 1:1, see Example 2) (average values ± SD; n=9).
Figure 4 shows the cellular proliferation values determined at different times (time zero T0, 2 days T2, 7 days T7, 10 days T10 and 15 days T15) on fibroblasts for just lysate and for the composition 1 (lysate: carrier weight ratio 1:1, see Example 1). The concentration of lysate in the composition is the same as is used for just lysate.

By control we mean the % proliferation of viable cells obtained in the absence of platelet lysate (LP) and of composition.

LP 1:20 and LP 1:40: % proliferation of viable cells obtained in the presence of platelet lysate (LP) at two different dilutions, 1:20 and 1:40, with culture medium lacking cellular growth factors.

Composition 1:20 and composition 1:40: % proliferation of viable cells obtained in the presence of platelet lysate (LP) carried in the composition at two different dilutions, 1:20 and 1:40, with culture medium lacking cellular growth factors.

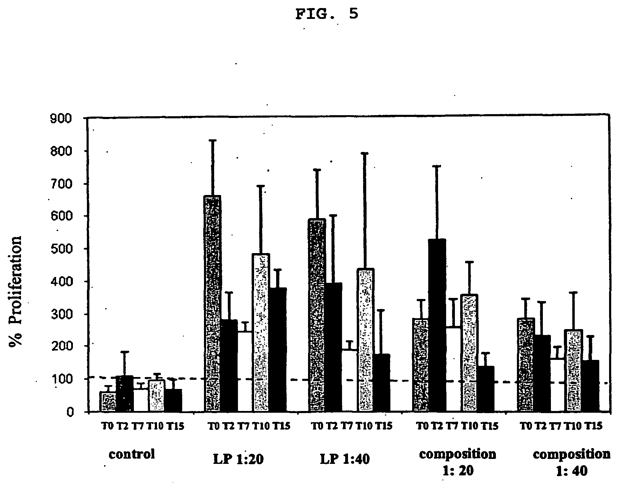

Figure 5 shows the cellular proliferation values determined at different times (time zero T0, 7 days T7, 10 days T10, 15 days T15 and 20 days T20) on a rabbit's corneal epithelial cellular lines for just lysate and for composition 2 (lysate:carrier weight ratio 1:1, see Example 2). The concentration of the lysate in the composition is the same as is used for just lysate.

By control we mean the % proliferation of viable cells obtained in the absence of platelet lysate (LP) and of composition.

LP 1:20 and LP 1:40: % proliferation of viable cells obtained in the presence of platelet lysate (LP) at two different dilutions, 1:20 and 1:40, with culture medium lacking cellular growth factors.

Composition 1:20 and composition 1:40: % proliferation of viable cells obtained in the presence of platelet lysate (LP) carried in the composition at two different dilutions, 1:20 and 1:40, with culture medium lacking cellular growth factors.

### DETAILED DESCRIPTION OF THE INVENTION

In a first object, the present invention concerns a pharmaceutical composition comprising a platelet lysate for use in the therapy and/or prophylaxis of mucositis.

In the present invention, said platelet lysate, abbreviated LP, can be "autologous", i.e. obtained from platelet-rich plasma (PRP) of the same patient for whom the composition of the invention is intended, or else "allogenic", i.e. formed from platelet-rich plasma (PRP) obtained from a subject known as a donor.

The use in therapy of autologous LP has the advantage of eliminating the risks linked to the transmission of disease. Moreover, the use of allogenic LP has the advantage of providing a large quantity of material having *standard* characteristics in terms of type and quantity of growth factors contained in it. To obtain autologous lysate, a peripheral venous blood sample suitably anti-coagulated, for example with acid citrate dextrose, is taken from the patient and subjected to centrifuging at 1000 rpm to separate the platelet-rich plasma (PRP) from the other corpuscular elements of blood like red or white blood cells. Then follows the rapid freezing of the PRP to a temperature of -80°C for 10 minutes and unfreezing of the sample without use of any exogenous substance, so as to cause the platelet membrane to break, determining the release by lysis into the plasma of the growth factors contained in the alpha granules of the platelets. On the other hand, in the case in which a sample of PRP collected through productive apheresis performed on donors through a cellular separator and then divided into aliquots of the volume of about 50 ml of platelets is used, it can be directly frozen and then unfrozen, according to the same methodologies described above to obtain autologous lysate, in this way preparing allogenic platelet lysate.

In both cases, however, the lysate obtained is rich in numerous growth factors.

Such a lysate has proven to surprisingly and unexpectedly possess much greater therapeutic properties, in promoting tissue repair both at mesenchymal and epithelial level, compared to known preparations comprising isolated growth factors.

Once prepared, the lysate is characterised through immunological dosage (ELISA) to evaluate the concentration of Human PDGF-AB: a particularly representative growth factor belonging to the family of human platelet growth factors.

The platelet lysate is then lyophilised to ensure its biological-therapeutic activity over time after having added suitable lyostabilizers, like, for example, trehalose, mannitol or glycine.

Such a platelet lysate can be applied to the site affected by mucositis, like, for example, the buccal, gastro-intestinal, nasal, vaginal or rectal mucosa or by vesical instillation.

In particular, such application can occur in the form of solution with possible addition of viscosifier agents like for example rubbers or aluminosilicates, sweeteners and/or flavourings. In terms of dosage, the application of the platelet lysate can occur in one of the forms indicated above and for an amount of between about 50-100 mg of lysate per cm² of mucosa.

In a further object, the present invention concerns a bioadhesive composition comprising a carrier and a platelet lysate for use as a medicament in mucositis prophylaxis or treatment.

By bioadhesive composition, we mean a composition capable of adhering to a physiological, human or animal surface, for example a tissue or a mucosa.

In the present invention, by "carrier" it is meant an aqueous solution of one or more mucoadhesive agents.

In particular, for the purposes of the present invention, by mucoadhesive agent we mean a compound or a mixture of compounds able to adhere to the mucosa, i.e. to the coating of the body cavities in communication with the outside through natural orifices. Such mucoadhesive agents, in particular, can be selected from polyacrylic acid (carbopol 974-P NF) or cross-linked polyacrylic acid like, for example, carbomer or polycarbophyl, or other derivatives of polyacrylic acids like, for example, polymethylmetacrylates; cellulose or derivatives of cellulose, like, for example, sodium carboxymethylcellulose, hydroxypropylcellulose, hydroxypropylmethylcellulose; other polysaccharides, like, for example, chitosan and its pharmaceutically acceptable salts, like, for example, chitosan glutamate, or, furthermore, polysaccharides like alginates, pregelatinised starches, pectins; glycosoaminoglycans, like, for example, hyaluronic acid, chondroitin sulphate; polyvinylpyrrolidone; gelatine; or mixtures of the mucoadhesive agents listed above.

The carrier is thus obtained by dissolution of one of the mucoadhesive agents indicated above in water, for example, distilled water or water in physiological solution, i.e. with 0.9% NaCl weight/volume, through agitation and subsequent addition of sweeteners and flavourings. Moreover, neutralising and/or buffer agents can be added, so that the pH is compatible with the physiological environment of the site of application.

For example, in the case in which the composition of the invention foresees buccal application, the pH values of the composition must be between 5.5 and 7.5 and, preferably between 6 and 7, even more preferably a pH value of 6.5.

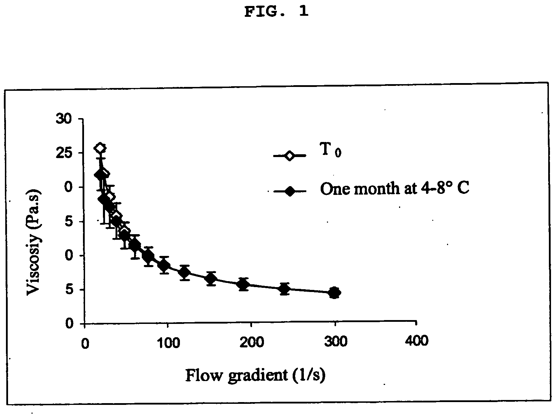

The carrier thus obtained can be conserved, by sterilisation or addition of preservatives known in the field, for up to 15-30 days at a temperature of 4-8°C maintaining its rheological characteristics, like viscosity, as illustrated in Figure 1 for the carrier relative to Example 1.

In particular, Figure 1 shows, as a function of the flow gradient, taken to be the ratio between the difference between the maximum and minimum flow speed in the thickness of the fluid and the thickness of the fluid itself and expressed in 1/s *(*P. Colombo et al, Principi di Tecnologie Farmaceutiche, Casa Editrice Ambrosiana, Milano, 2004*),* the viscosity, expressed in Pascals (Pa), for the carrier made according to the present invention, at time zero, i.e. just prepared, and one month after its preparation.

Before being mixed with the other components of the composition of the invention, the carrier, prepared as described above, is sterilised in an autoclave, according to methodologies known to the man skilled in the art.

The mucoadhesive composition for use of the invention comprises, as stated above, in addition to the carrier containing the mucoadhesive agent, a platelet lysate (LP).

Said lysate, in particular, is the lysate obtained according to what has been described above and is mixed with the "carrier" obtained as detailed above.

The mixing of the carrier with the platelet lysate, in particular, is carried out so as to obtain a homogeneous composition.

More specifically, the for use composition of the invention comprises platelet lysate and the carrier, in a weight ratio of between 1:6 and 6:1. In a preferred aspect, such a weight ratio is between 1:2 and 2:1 and, even more preferably, it is 1:1.

The composition for use of the invention has rheological properties, like viscosity, which allow easy application to the mucosae, but, at the same time, a surprising and unexpected mechanical strength.

In this way, the composition has demonstrated that it remains for an advantageously long time at the site on which it has to carry out the therapeutic and/or prophylactic activity.

Consequently, the number of applications on the site affected by mucositis or corneal lesion can be advantageously reduced, whilst still ensuring effectiveness and speed in acting therapeutically.

For example, in the case in which the composition of the invention foresees a buccal application, the pH values of the composition must, as mentioned above, be between 5.5 and 7.5, preferably between 6 and 7, a pH value of 6.5 being even more preferable (see Table I shown below).

The respective viscosity values measured at flow gradient equal to 50 s⁻¹ (see, in particular, P. Colombo et al, Principi di Tecnologie Farmaceutiche, Casa Editrice Ambrosiana, Milano, 2004*)* of said compositions are between 1 and 8 Pa.s, preferably between 1.5 and 6 Pa.s and even more preferably they are between 2 and 4.5 Pa.s (see Table I below).

**Table 1**

| pH | Apparent viscosity (Pa.s) |
|---|---|
| 5.5 - 7,5 | 1 - 8 |
| 6 - 7 | 1.5 - 6 |
| 6.5 | 2 - 4.5 |

One of the advantages of the composition of the invention for use is represented by its properties of mucoadhesion, i.e. the ability to interact with the mucus and/or the mucosa of application so as to remain for an extended time in the site of application.

Said properties are measured by evaluating the detachment force, i.e. the force necessary to detach a layer of the composition by a mucin gel. The test foresees using a commercial apparatus (TA XTPlus) or another analogous apparatus for measuring the traction force. In brief, in the adopted test, widely described in literature, a layer of composition is positioned on a surface of inert material and arranged in contact with a commercial mucin solution mimicking the surface of the mucosa (the biological substrate). Then the force necessary to detach the composition from the biological substrate is measured (see, for a general reference, S. Rossi et al., Chitosan ascorbate: a chitosan salt with improved penetration enhancement properties. Pharm. Dev. Technol. 13 (6), (2008), 513-21).

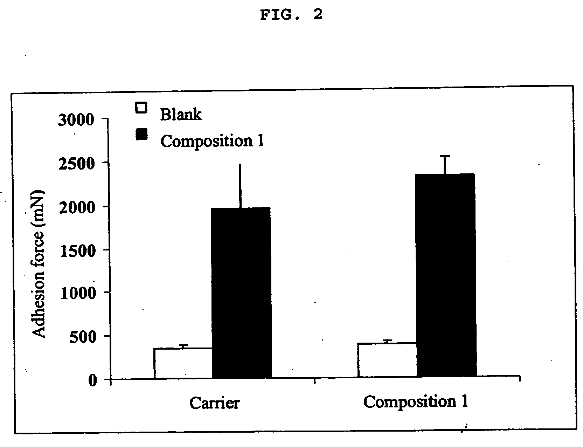

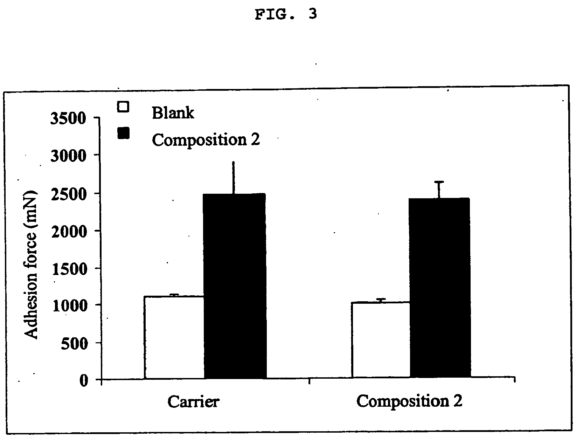

The data relative to the compositions of Examples 1 and 2 are illustrated in Figures 2 and 3.

In particular, by "maximum adhesion force" we mean the maximum force recorded during the detachment experiment of the composition from the biological substrate.

From Figures 2 and 3, it can clearly be seen that both the carrier and the compositions comprising such a carrier are characterised by greater detachment force values in the presence of mucin compared to the blank, i.e. to measurements carried out on the composition using buffer in the test instead of the biological substrate.

The experimental data, therefore, demonstrates the advantageous and unexpected ability of the compositions to adhere to the biological substrate.

Indeed, the lack of significant differences between the maximum force values observed for just the carrier with respect to the composition of the invention indicates that the mixing of the carrier with the platelet lysate (LP) surprisingly does not affect the ability of the mucoadhesive agent to carry out its own activity.

Moreover, the compositions for use of the invention unexpectedly demonstrate a further advantage, since they are able to release the growth factors present in the platelet lysate without the action promoting cellular proliferation being compromised.

The compositions for use of the present invention, in particular, can be prepared in the form of a gel, which is a semi-solid system, as described in Examples 1 and 2; as a film, obtained by drying from a gel from; or as a "sponge like" system, obtained by lyophilization of a gel form. Both the film form and the "sponge like" form are intended for direct local application on the lesions. Alternatively, the composition can also be dried in the form of particulate or micro-particulate dispersions, for example by nebulisation, representing a powder to be applied directly or after pre-suspension in a suitable medium.

Such dispersions, in particular, increase the contact surface between therapeutic agent and mucosa to be treated.

In particular, the film, "sponge-like", and micro-particulate and particulate forms conserve the adhesive properties of the gel from which they are obtained.

*Films, sponge like* systems and particulate or micro-particulate systems can be obtained according to methods commonly known to men skilled in the art (see, for a general reference, P. Colombo et al, Principi di Tecnologie Farmaceutiche, Casa Editrice Ambrosiana, Milano, 2004*)*

In addition, the mucoadhesive composition for use of the present invention can be prepared, for example in a hospital facility, shortly before application to the patient, for example by rehydration of the composition of the invention prepared in lyophilized form with a suitable agent.

The mucoadhesive composition for use according to the invention can also comprise pharmaceutically acceptable technological agents like emollients, sweeteners, like, for example, saccharin, flavourings, like mint, strawberry or liquorice flavour, pH modifiers, like NaOH solutions or buffers, preservatives or viscosifiers, as known to the skilled in the art.

With regard to the therapeutic doses of the compositions for use of the invention, they can be applied, for example, in quantities of between 100-200 mg comprising platelet lysate and carrier in a 1:1 weight ratio per cm² of mucosa.

According to a further aspect, the present invention concerns the compositions of the invention comprising a carrier and a platelet lysate for use as a medicament in mucositis prophylaxis or treatment.

More specifically, said compositions can be applied onto mucosae for the prophylaxis and/or therapy of mucositis.

Mucositis is an inflammation that affects the mucosae and, as described above, it can have a different etiogenesis, including anti-tumour radio- and chemotherapy.

The seriousness of mucositis can be evaluated in grades, from I to IV, according to the extension and the symptoms, as follows:

| **Mucositis grade** | **Symptoms** |
|---|---|
| I | Erithema of the mucosa |
| II | Patchy or pseudomembranous ulceration |
| III | confluent or pseudo-membranous ulcerations; bleeding with small trauma |
| IV | Necrosis of the tissue with significant spontaneous bleeding; consequences placing life at risk |

Corneal lesions, on the other hand, can be caused by foreign or intraocular bodies and are particularly harmful if not recognised and not cured. Moreover, if no therapeutic intervention is made, there can easily be secondary infections.

In these cases, the application of the compositions of the invention can take place on mucositis of the buccal, gastro-intestinal, nasal, vaginal and rectal mucosa, by vesical instillation, as well as, in the case of corneal lesions, on conjunctival mucosa.

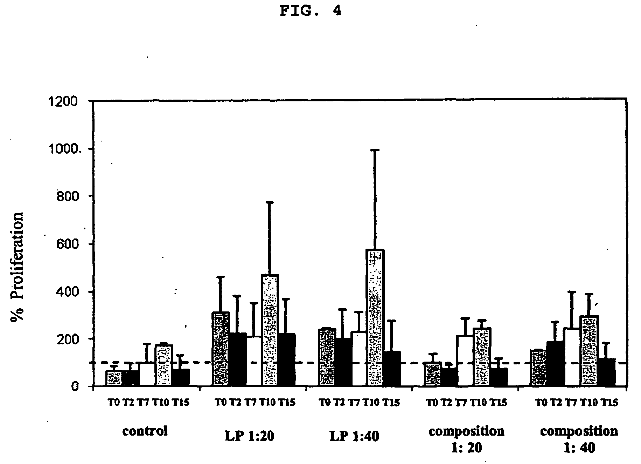

The therapeutic properties of the compositions of the invention can be checked "*in-vitro*" through cellular proliferation measurements made on different cellular lines; in the present case a rabbit's fibroblasts and corneal epithelial lines have been used, as illustrated in Figures 4 - 5.

In particular, said cellular proliferation measurements are made by placing the suitably diluted composition in contact for 24 hours with the cells grown in wells. Then the number of live cells is evaluated through a cell survival test (neutral red (NR) assay (Tox Kit 4, Sigma-Aldrich, Milano, Italy) (*for a general reference, see, for example,* M.C. Bonferoni et al., Chitosan citrate as multifunctional polymer for vaginal delivery. Evaluation of penetration enhancement and peptidase inhibition properties. Eur. J. Pharm. Sci. 33, (2008) 166-176*)*.

The present invention is described further hereafter with examples given purely for illustrative purposes of the different aspects of the making of the compositions object of the invention and they are not intended to limit the invention in any way.

### Example 1

### Preparation of composition 1

### a) preparation of the carrier

A gel based upon polyacrylic acid (carbopol 974-P NF) is prepared having the following composition:
- carbopol 5% (p/p)
- saccharin 0.2% (p/p)
- flavourings 0.2% (p/p)
- NaOH 4 N up to pH 7.

In particular, the saccharin is dissolved in a physiological solution (0,9% weight/volume of NaCl) through magnetic agitation. Carbopol is added and the mixture is kept under agitation until complete hydration (about 12 hours). When hydration is complete, a flavouring is added, for example mint, liquorice or strawberry. The formulation is then buffered to pH 7 through the use of a solution of NaOH 4 N in order to obtain the gelification of the carbopol and thus a formulation having a pH compatible with the mucosa of the oral cavity.

The carrier thus prepared is then poured into a heat-resistant glass bottle, the empty space at the top is filled with nitrogen and it is subjected to sterilisation with damp heat in an autoclave for 15 minutes at a temperature of 121°C,

### b) preparation of composition 1

The carrier obtained in step a) is then mixed with platelet lysate in a 1:1 weight ratio obtaining a final carbopol concentration of about 2.5% by weight of the total of the composition.

### Example 2

### Preparation of composition 2

### a) preparation of the carrier for composition 2

A gel based upon chitosan and hydroxypropylmethylcellulose (HPMC) was prepared with the following composition:
- chitosan glutamate 6% (p/p)
- HPMC 2% (p/p)

The hydroxypropylmethylcellulose is hydrated with a minimum amount of distilled water.

Chitosan glutamate is added until a concentration of 6% p/p is obtained and the whole thing is brought up to volume with water and kept under mild agitation until the complete hydration of the polymers.

The pH of the carrier thus prepared is equal to 5.5.

The gel is then poured into a heat-resistant glass bottle, the empty space at the top is filled with nitrogen and it is subjected to sterilisation with damp heat in an autoclave for 15 minutes at 121°C.

### b) preparation of composition 2

The carrier obtained in step a) is then mixed with platelet lysate in a 1:1 weight ratio up to a final concentration of chitosan glutamate of 3% (p/p) and of HPMC of 1% (p/p).

### Example 3

### Characteristics of composition 1

The composition 1 has a pH value equal to about 7 and rheological properties suitable for application in the oral cavity: apparent viscosity at 50 s⁻¹ equal to about 4.5 Pa.s.

The composition has properties of mucoadhesion as illustrated in Figure 2.

The composition maintains the ability to promote cellular proliferation even after conservation at a temperature of 4-8°C for 10-15 days as represented in Figure 4.

The composition 1 in a preliminary *in vivo* study proved effective in the treatment of mucositis in five patients suffering from mucositis of the oral cavity of grade III-IV.

In particular, in 4 such patients the response involved a recovery of the integrity of the damaged of between 50% and 100% and only in one case was it less than 50%. In just one patient with grade I mucositis was there no response.

No patients suffered local infections as a result of application of the lysate in mucoadhesive carrier.

### Example 4

### Characteristics of composition 2

Composition 2 has rheological properties suitable or application in the oral cavity: apparent viscosity 50 s⁻¹ equal to about 3 Pa.s.

The composition has properties of mucoadhesion as illustrated in Figure 3.

The composition has demonstrated that it maintains the ability to promote cellular proliferation even after storage at a temperature of 4-8 °C for 10-15 days as represented in Figure 5.

## Claims

1. A pharmaceutical composition comprising a platelet lysate and a carrier, wherein said carrier comprises an aqueous solution of one or more mucoadhesive agents, for use as a medicament in mucositis prophylaxis or treatment.

2. Pharmaceutical composition for use according to claim 1, wherein said mucoadhesive agent is selected from the group comprising: polyacrylic acid and its derivatives; glucosaminoglycans and its derivatives; polyvinylpyrrolidone; gelatine; chitosan and its salts; alginates; pregelatinised starches; pectins; and their mixtures.

3. Pharmaceutical composition for use according to claim 2, wherein said derivatives of polyacrylic acid comprise carbomer, polycarbophyl and polydimethylmetacrylates.

4. Pharmaceutical composition for use according to claim 2, wherein said glucosaminoglycans comprise chondroitin sulphate and hyaluronic acid.

5. Pharmaceutical composition for use according to claim 2, wherein said derivatives of cellulose comprise sodium carboxymethylcellulose, hydroxycellulose and hydroxypropylmethylcellulose.

6. Pharmaceutical composition for use according to any of the preceding claims, wherein is in the form of a semisolid system, of film obtained by drying a semisolid system, of a lyophilized system, of particle or microparticle dispersions.

7. Pharmaceutical composition for use according to claim 6, wherein said lyophilized system is of the "sponge-like" type.

8. Pharmaceutical composition for use according to any of the preceding claims, wherein it has a pH of between 5.5 and 7.5 and, preferably between 6 and 7, even more preferably a pH value of about 6.5.

9. Pharmaceutical composition for use according to claim 8, wherein it has a pH of between 5.5 and 7.5 and viscosity between 1 and 8 Pa.s, preferably having a pH comprised between 6 and 7 and viscosity between 1.5 and 6 Pa.s and even more preferably having pH of about 6.5 and viscosity between 2 and 4.5 Pa.s.

10. Pharmaceutical composition for use according to any preceding claim, wherein said platelet lysate and said carrier are contained in a weight ratio of between 1:6 and 6:1, preferably of between 1:2 and 2:1 and, even more preferably, of 1:1.

11. Pharmaceutical composition for use according to any of the preceding claims, wherein it comprises viscosifiers and/or sweeteners and/or flavourings.

## Patentansprüche

1. Pharmazeutische Zusammensetzung umfassend ein Thrombozytenlysat und einen Träger, wobei der Träger eine wässrige Lösung ein oder mehrerer muco-adhäsiver Stoffe umfasst zur Verwendung als ein Medikament für die Prophylaxe oder Behandlung von Mucositis.

2. Pharmazeutische Zusammensetzung zur Verwendung nach Anspruch 1, in welcher der muco-adhäsive Stoff ausgewählt ist aus der Gruppe umfassend: Polyacrylsäure und ihre Derivate; Glycosaminoglykane und deren Derivate; Polyvinylpyrrolidon; Gelantine; Chitosan und seine Salze; Alginate; vorgelatinierte Stärken; Pektine; und ihre Mischungen.

3. Pharmazeutische Zusammensetzung zur Verwendung nach Anspruch 2, in welcher die Derivate der Polyacrylsäure umfassen Carbomer, Polycarbophyl- und Polydimethylmetacrylate.

4. Pharmazeutische Zusammensetzung zur Verwendung nach Anspruch 2, in welcher die Glycosaminoglykane umfassen Chondroitinsulfat und Hyaluronsäure.

5. Pharmazeutische Zusammensetzung zur Verwendung nach Anspruch 2, in welcher die Derivate der Zellulose umfassen Natrium-Carboxymethylcellulose, Hydroxycellulose und Hydroxypropylmethylcellulose umfassen.

6. Pharmazeutische Zusammensetzung zur Verwendung nach einem der vorhergehenden Ansprüche, wobei diese vorliegt in Form eines halbfesten Systems, eines Films, der durch Trocknen eines halbfesten Systems erhalten wird, eines lyophilisierten Systems, aus Teilchen- oder Mikroteilchendispersionen.

7. Pharmazeutische Zusammensetzung zur Verwendung nach Anspruch 6, in welcher das lyophilisierte System als schwammartiger Typus vorliegt.

8. Pharmazeutische Zusammensetzung zur Verwendung nach einem der vorhergehenden Ansprüche, in welcher diese einen pH von zwischen 5,5 und 7,5 und vorzugsweise zwischen 6 und 7, ganz bevorzugt einen pH-Wert von etwa 6,5 hat.

9. Pharmazeutischen Zusammensetzung zur Verwendung nach Anspruch 8, in welcher diese einen pH zwischen 5,5 und 7,5 und eine Viskosität zwischen 1 und 8 Pa s, vorzugweise mit einem pH zwischen 6 und 7 und einer Viskosität zwischen 1,5 und 6 Pa s und ganz bevorzugt mit einem pH von etwa 6,5 und einer Viskosität zwischen 2 und 4,5 Pa s hat.

10. Pharmazeutische Zusammensetzung zur Verwendung nach einem vorhergehenden Anspruch, in welcher das Thrombozytenlysat und der Träger in einem Gewichtsverhältnis von zwischen 1:6 und 6:1, vorzugsweise zwischen 1:2 und 2:1 und ganz bevorzugt 1:1 enthalten sind.

11. Pharmazeutische Zusammensetzung zur Verwendung nach einem der vorhergehenden Ansprüche, in welcher diese Verdickungsmittel und/oder Süßstoffe und/oder Aromastoffe umfasst.

## Revendications

1. Composition pharmaceutique comprenant un lysat plaquettaire et un support, dans laquelle ledit support comprend une solution aqueuse d'un ou de plusieurs agents mucoadhésifs, pour une utilisation en tant que médicament dans la prophylaxie ou le traitement de la mucosité.

2. Composition pharmaceutique pour son utilisation selon la revendication 1, dans laquelle ledit agent mucoadhésif est choisi dans le groupe constitué par : l'acide polyacrylique et ses dérivés ; les glucosaminoglycanes et leurs dérivés ; la polyvinylpyrrolidone ; la gélatine ; le chitosane et ses sels ; les alginates ; les amidons prégélatinisés ; les pectines ; et leurs mélanges.

3. Composition pharmaceutique pour son utilisation selon la revendication 2, dans laquelle lesdits dérivés d'acide polyacrylique comprennent le carbomère, le polycarbophile et les méthacrylates de polydiméthyle.

4. Composition pharmaceutique pour son utilisation selon la revendication 2, dans laquelle lesdits glucosaminoglycanes comprennent le sulfate de chondroïtine et l'acide hyaluronique.

5. Composition pharmaceutique pour son utilisation selon la revendication 2, dans laquelle lesdits dérivés de cellulose comprennent la carboxyméthylcellulose sodique, l'hydroxycellulose et l'hydroxypropylméthylcellulose.

6. Composition pharmaceutique pour son utilisation selon l'une quelconque des revendications précédentes, dans laquelle elle est sous la forme d'un système semi-solide, d'un film obtenu par séchage d'un système semi-solide, d'un système lyophilisé, de dispersions de particules ou de microparticules.

7. Composition pharmaceutique pour son utilisation selon la revendication 6, dans laquelle ledit système lyophilisé est de type "spongieux".

8. Composition pharmaceutique pour son utilisation selon l'une quelconque des revendications précédentes, dans laquelle elle présente un pH compris entre 5,5 et 7,5 et, de préférence entre 6 et 7, plus préférentiellement encore une valeur de pH d'environ 6,5.

9. Composition pharmaceutique pour son utilisation selon la revendication 8, dans laquelle elle présente un pH compris entre 5,5 et 7,5 et une viscosité comprise entre 1 et 8 Pa.s, de préférence présentant un pH compris entre 6 et 7 et une viscosité comprise entre 1,5 et 6 Pa.s et plus préférentiellement encore présentant un pH d'environ 6,5 et une viscosité comprise entre 2 et 4,5 Pa.s.

10. Composition pharmaceutique pour son utilisation selon l'une quelconque des revendications précédentes, dans laquelle ledit lysat plaquettaire et ledit support sont contenus dans un rapport pondéral compris entre 1:6 et 6:1, de préférence entre 1:2 et 2:1 et, plus préférentiellement encore, de 1:1.

11. Composition pharmaceutique pour son utilisation selon l'une quelconque des revendications précédentes, dans laquelle elle comprend des viscosifiants et/ou des édulcorants et/ou des arômes.
